# EUROPEAN PATENT SPECIFICATION

(11) **EP 2 924 142 B1**
(45) Date of publication and mention of the grant of the patent: **16.11.2016**
(21) Application number: 15000965.2
(22) Date of filing: 19.10.2012
(51) Int. Cl.: C23C 14/02, C23C 14/06, C23C 14/34, C23C 14/35, C23C 14/50, C23C 28/00, H01J 37/32, H01J 37/34, A61L 27/30

(54) **A NANO-MULTILAYER FILM**
NANO-MEHRSCHICHTFILM
FILM NANO-MULTICOUCHE

(30) Priority: 15.05.2012 CN 201210151152; 15.05.2012 CN 201220218296 U
(43) Date of publication of application: 30.09.2015
(62) Divisional of application: 12250163.8
(73) Proprietor: ZhongAo HuiCheng Technology Co. Ltd., Beijing 100076 (CN)
(72) Inventor: Jin, Gong, Beijing 100076 (CN); Tu, Jiangping, Beijing 100076 (CN); Li, Lingling, Beijing 100076 (CN); Wang, Meina, Beijing 100076 (CN); Wang, Gang, Beijing 100076 (CN)
(74) Representative: Atkinson, Ralph

(56) References cited:
- CN-A- 102 247 620
- US-A1- 2007 111 003
- VOEVODIN A A ET AL: "Design of a Ti/TiC/DLC functionally gradient coating based on studies of structural transitions in Ti-C thin films", THIN SOLID FILMS, ELSEVIER-SEQUOIA S.A. LAUSANNE, CH, vol. 298, no. 1-2, 20 April 1997 (1997-04-20), pages 107-115, XP004125924, ISSN: 0040-6090, DOI: 10.1016/S0040-6090(96)09145-6
- GIOTI M ET AL: "Stress relaxation and stability in thick amorphous carbon films deposited in layer structure", APPLIED PHYSICS LETTERS, AIP, AMERICAN INSTITUTE OF PHYSICS, MELVILLE, NY, US, vol. 73, no. 2, 13 July 1998 (1998-07-13), pages 184-186, XP012021375, ISSN: 0003-6951, DOI: 10.1063/1.121749

## Description

The present invention relates to a nano-multilayer film.

Currently, the vacuum magnetron sputtering technology has been extensively applied in various fields. One of the applications is coating the surface of a substrate, e.g. the surfaces of such substrates as medical surgical equipment, human implantation medical materials and engineering tools, with the diamond-like carbon (DLC) by making use of the vacuum magnetron sputtering technology, to significantly increase hardness and wear resistance of the substrate.

However, the diamond-like carbon, having a bigger internal stress, has a binding force not high with metal or alloy materials, and is prone to rupture or peeling under the action of higher load or load impact; especially for the human implantation product, the peeling of diamond-like carbon may produce debris, which will aggravate wear of the implantation product and reduce the service life of the product.

In order to resolve the above problems, the present research provides a nano-multilayer film, which can increase the wear resistance of the substrate, increase the binding force with the substrate, and contribute to improvement of lubricity at the same time. For example, the presented carbon/titanium carbide nano-multilayer film is coated onto the surface of the metal articular head and acetabular cup of the human-implantation product, making the articular head and acetabular cup have excellent wear resistance and good biocompatibility, as well as good lubricity, significantly increasing the service life of the artificial joint in the human body.

However, there is a very high requirement for the power technology when processing the nano-multilayer film by making use of the current magnetron sputtering coating device and, due to the limitation of the current industrial system, it is very difficult to achieve precise control on the industrialized scale and to realize industrialized production of the multilayer film.

An apparatus of the prior art for physical vapour deposition is disclosed in German patent publication DE 10 2008 062 332 A1. The apparatus is equipped with vaporisers of differing compositions, and has a segregated treatment chamber such that substrates are only subjected to deposition from one vaporiser at a time.

A multilayer amorphous carbon film, and a method of achieving stress relaxation in thick amorphous carbon films is disclosed in Appl. Phys. Lett. 73, 184 (1998), NPL number XP012021375. Amorphous carbon films are deposited by sputtering with alternating positive and negative bias voltages to obtain layers alternating between being rich in *sp*² sites and *sp*³ sites.

An alternative multilayer amorphous carbon film is disclosed in United States patent publication US 2007/0111003 A1. The multilayer film comprises first an adhesive layer on a substrate. Above the adhesive layer are multiple layers of doped diamond-like carbon, above which is a layer of undoped diamond-like carbon. The amount of dopant decreases from the lower to the top layer.

A further multilayer film is disclosed in Chinese patent publication CN 102247620 A. This film includes alternating layers of carbon, and a mixture of carbon and titanium carbide. The film is applied by a process of magnetron sputtering using two graphite targets and two titanium targets arranged around a rotating gantry.

A film with a gradually changing composition is disclosed in Thin Solid Films vol. 298, nr. 1-2, pp. 107-115 (1998), NPL number XP004125924. An initial layer of *α*-Ti is applied to a substrate. Above this, a plurality of layers of titanium carbide in the form TiₓC_{y} are applied, with x, the titanium content, decreasing from 0.90 to 0.10 and y, the carbon content, increasing from 0.10 to 0.90. Finally a layer of diamond-like carbon is applied. The resulting film resists brittle failure tests.

A purpose of the present invention is to at least improve upon the proposals set forth in the prior art in terms of the nano-multilayer film itself.

Thus according to an aspect of the present invention, there is provided a nano-multilayer film prepared by magnetron sputtering coating, comprising a composite layer, which is a multilayer structure with a quasi-graphite layer that includes titanium, and a diamond-like carbon layer alternately laminated, where the *sp*² content in the quasi-graphite layer is about 60 % and the *sp*³ content in the diamond-like carbon layer is about 70 %, and a diamond-like carbon top-layer film on the composite layer. The nano-multilayer film is characterized in that it further comprises a transition layer in combination with a substrate below the composite layer, which transition layer includes a titanium film and a mixed layer of titanium carbide and quasi-graphite on the titanium film, and in the direction from the substrate to the diamond-like carbon top-layer film, the mass percentage of Ti in the mixed layer of titanium carbide and quasi-graphite is gradually decreased, while the mass percentage of C is gradually increased.

Optionally, the transition layer has a thickness of 300-500 nm.

Optionally, the composite layer has a thickness of 1600-3300 nm.

Optionally, the diamond-like carbon top-layer film has a thickness of 100-150 nm.

Optionally, the mass percentage of C in the nano-multilayer film is 70-97.6 %, and the mass percentage of Ti is 2.4-30 %.

Compared with the prior art, the magnetron sputtering coating device equally divides the rotating stand into two mutually independent areas by arranging a baffle on the rotating stand. Because both ends of the baffle are beyond both ends of the sputtering cathode in the direction perpendicular to the rotating stand, the product to be coated at different areas, adapted to the position set for the sputtering cathode and at a certain position in the rotation of the rotating stand, only accepts coating from the first sputtering cathode and the second sputtering cathode, respectively, or only from the first sputtering cathode. Thus the nano-multilayer film is prepared by the rotation of the rotating stand. The device is simple in structure and process control, and realizes preparation of the nano-multilayer film, suitable for industrialization.

By making use of the magnetron sputtering coating device and method, through controlling the working current and voltage at different sputtering stage, the nano-multilayer film is formed, including the transition layer, the composite layer of alternately laminated diamond-like carbon and quasi-graphite layers, and the diamond-like carbon top-layer film, having better binding force, lubricity and wear resistance.

The above and/or additional aspects and advantages of the present invention will become obvious and comprehensible from the following description of the examples with reference to the drawings.
Fig. 1 is a stereoscopic structural schematic view of the magnetron sputtering coating device for use in preparing the nano-multilayer film of the present invention.
Fig. 2 is a top schematic view of the magnetron sputtering coating device as shown in Fig. 1.
Fig. 3 is a top schematic view of an alternative magnetron sputtering coating device not forming part of the present invention.
Fig. 4 is a sectional structural schematic view of the nano-multilayer film in combination with the metal material according to the example of the present invention.

For better understanding the technical solution and beneficial effects of the present invention, the examples of the present invention will be described in detail below. The examples described below with reference to the drawings are exemplary only for explanation rather than restriction of the present invention.

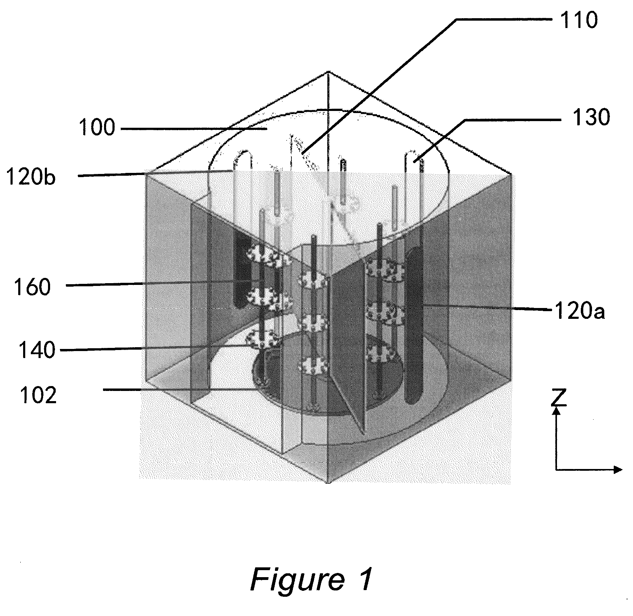

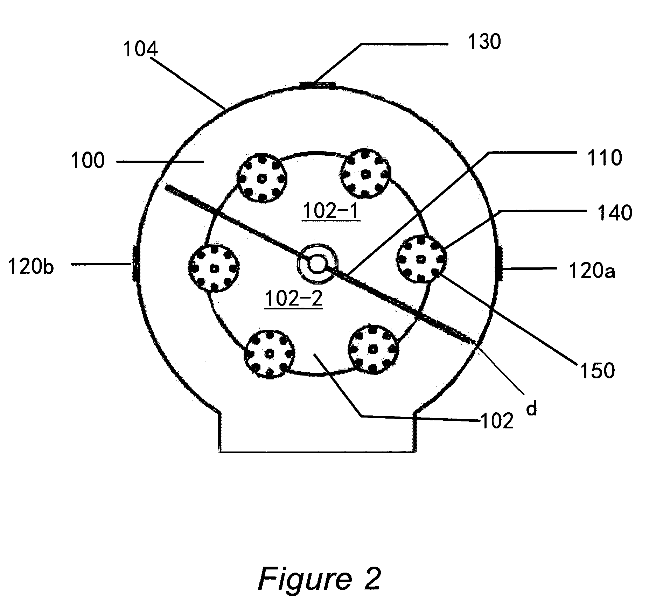

As shown in Fig. 1 and Fig. 2, Fig. 1 and Fig. 2 is a stereoscopic and top structural schematic view of the magnetron sputtering coating device for use in preparing the nano-multilayer film of the present invention. The magnetron sputtering coating device in the example includes a deposition chamber 100, a rotating stand 102, a sputtering cathode, and a work steady 140, as well as a first rotation system (not shown in the drawing) for driving the rotating stand 102 to rotate around a central axis of the rotating stand; the device certainly further includes other necessary parts, such as a heating device, a temperature control system, a cooling water circulation system, and a power supply system electrically connected with the sputtering cathode (not shown in the drawing).

In the example, the sputtering cathode includes two first sputtering cathodes 120a and 120b and one second sputtering cathode 130, which are arranged on the circumference 104 concentric with the rotating stand 102; the circumference where the sputtering cathode is located can either be an actual part such as the inner wall of the deposition chamber 100, or be a virtual circumference such as any position between the rotating stand and the deposition chamber, where the two first sputtering cathodes 120a and 120b are arranged relatively parallel to each other and equally divide the circumference 104, while the second sputtering cathode 130 equally divides the arc between the two first sputtering cathodes 120a and 120b; that is, the arc between the two first sputtering cathodes 120a and 120b is substantially of 180°, while the arc between the second sputtering cathode 130 and the first sputtering cathodes 120a and 120b is substantially of 90°; the first sputtering cathodes 120a and 120b can be a sputtering cathode of a certain chemical element, while the second sputtering cathode can be a sputtering cathode of another chemical element. The sputtering cathode material can be selected according to the specific product needing to be coated; to prepare the nano-multilayer film of the present invention, the first sputtering cathode is a graphite target while the second sputtering cathode a titanium target.

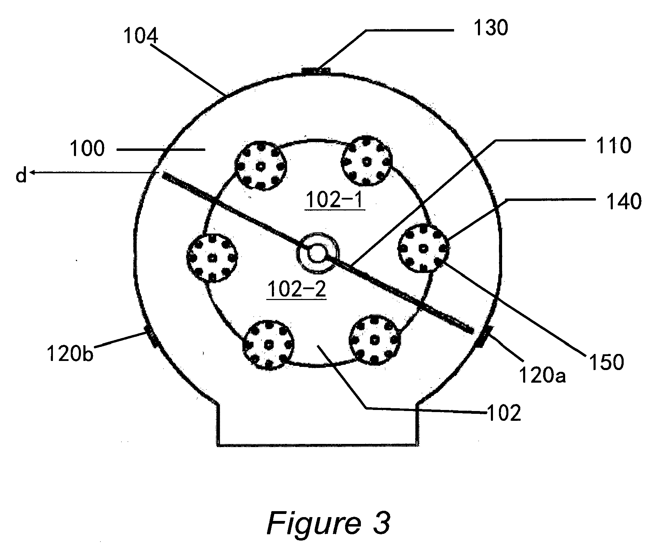

In other examples not forming part of the present invention, the two first sputtering cathodes and one second sputtering cathode can also be arranged on the circumference at intervals at other angles; for example, the arc (superior arc) between the two first sputtering cathodes 120a and 120b is of 180-240°, while the one second sputtering cathode equally divides the arc; therefore, the arc between the second sputtering cathode and the first sputtering cathode can be of 90-120°, as shown in Fig. 3, while the arc between the second sputtering cathode 130 and the first sputtering cathodes 120a and 120b is of 120°.

In the example the rotating stand 102 is a circular platform, on which is fixed a baffle 110; the baffle 110, being preferably a straight plate, can be made of titanium, aluminium, stainless steel and the like or the combination thereof; the baffle 110 goes through the diameter of the rotating stand and is arranged perpendicular to the rotating stand 102, dividing the rotating stand 102 into two mutually independent areas 102-1 and 102-2; both ends of the baffle 110, in the direction perpendicular to the rotating stand (the direction of the Z axis), are beyond both ends of the sputtering cathodes 120a, 120b and 130; therefore, the baffle blocks the other face of the sputtering cathode in a certain area, making the area only accept the coating of the sputtering cathode opposing the area; more preferably, for achieving a better blocking effect, the baffle has a width bigger than the diameter of the rotating stand, where the width refers to the length of the baffle going through the rotating stand in the diameter direction; more preferably, the distance d between the baffle and the circumference where the sputtering cathode is located is 2-10 cm.

The rotating stand 102 is provided with a first rotation system (not shown in the drawing) for driving the rotating stand to rotate around a central axis of the rotating stand; that is, the rotating stand and the baffle along with the work steady rotate around the central axis of the rotating stand. Under the condition of the above arrangement of the three sputtering cathodes, when the rotating stand rotates to any position, e.g. the position as shown in Fig. 2, with the blocking of the baffle 102, an area 102-1 of the rotating stand is opposed to the first sputtering cathode 120a (e.g. the graphite target) and the second sputtering cathode 130 (the titanium target), enabling the product (or the substrate) to be coated in the area 102-1 with a film of carbon:titanium carbide (a mixed layer of carbon with titanium carbide); while another area 102-2 of the rotating stand is opposed to the first sputtering cathode 120b (the graphite target), thus making the product in the area 102-2 coated with the carbon film; with rotation of the rotating stand, the product in different areas will be coated alternately with the carbon:titanium carbide film and the carbon film, thus realizing the coating of the product with the nano-multilayer film. The thickness of a single layer can be controlled by adjustment of the rotational speed of the rotating stand. The device is simple in structure and process control, and realizes preparation of the nano-multilayer film, suitable for industrialization.

In other examples not forming part of the present invention, the baffle 110 can also be arranged perpendicularly at other positions of the rotating stand, or be a bent plate or any other baffle that divides the rotating stand into two mutually independent areas.

In the example the device further has a second rotation system for driving the work steady to rotate around the central axis of the work steady; that is, the work steady can rotate on its axis. Multiple work steadies 140 are arranged on the rotating stand 102 through the support rod 160, and the same support rod 160 can be provided at intervals with multiple work steadies 140 to improve the processing efficiency; the work steady 140 is used to receive the substrate (or product) 150 to be processed, which can be uniformly arranged on the circumference of the work steady 140. With the rotation of the work steady on its axis, the coating on each of the products to be coated on the work steady can have good uniformity.

In another example, there are four sputtering cathodes (not shown in the drawing) arranged around the rotating stand; that is, the sputtering cathode includes two first sputtering cathodes and two second sputtering cathodes, the two first sputtering cathodes arranged opposite to each other, the two second sputtering cathodes arranged opposite to each other, the four sputtering cathodes equally dividing the circumference. However, during the preparation of the multilayer film, one of the second sputtering cathodes does not work; that is, the corresponding parameters such as the target current and voltage are set for the two first sputtering cathodes and one of the second sputtering cathodes instead of for the other second sputtering cathode, which is not used for sputtering and in an idle state. In the example, although four sputtering cathodes are arranged, one of them is not used for sputtering.

In the present invention, the substrate to be coated can either be human implantation equipment such as bone articular head or acetabular cup, or be other substrates such as engineering items, the substrate being made of metal or alloy materials or other materials.

What is described above is a detailed description of the magnetron sputtering coating device for use in the preparation of the nano-multilayer film of the present invention. During the preparation, as required specifically, each processing parameter is set for the preparation of the nano-multilayer film. For this, the a method of coating the substrate is provided by making use of any of the above magnetron sputtering coating devices, the method comprising three sputtering stages.

In a first sputtering stage, the working current of two first sputtering cathodes is set to zero. A second sputtering cathode is set to have a working current of first predetermined current value and to be at constant voltage mode, the working bias voltage is kept at first predetermined bias voltage and then the sputtering of a first predetermined duration is performed. Then, all sputtering cathodes are at constant voltage mode, the working bias voltage is kept at a second predetermined bias voltage. working current of the two first sputtering cathodes, starting from an initial working current I1, is increased by ΔI1 at intervals of a first time interval T1, until reaching a second predetermined current value; the working current of the one second sputtering cathode, starting from the first predetermined current value, is decreased by ΔI2 at intervals of a second time interval T2, until reaching a third predetermined current value.

In a second sputtering stage, all sputtering cathodes are at constant voltage mode, the working bias voltage is kept at the second predetermined bias voltage. the working current of the second sputtering cathode is set to and kept at a fourth predetermined current value, the working current of the first sputtering cathode is kept at the second predetermined current value or is set to and kept at a fifth predetermined current value, and then the sputtering of a second predetermined duration is performed.

In a third sputtering stage, working bias voltage is kept at the second predetermined bias voltage, the working current and voltage of the second sputtering cathode are set to zero, the first sputtering cathode is kept at the working current of the second sputtering stage or is set to and kept at a sixth predetermined current value, and is kept at the constant voltage mode, and then the sputtering of a third predetermined duration is performed.

In the examples of the preparation method for the nano-multilayer film of present invention, three sputtering cathodes, i.e. two first sputtering cathodes and one second sputtering cathode, are used for the preparation; in the example further including the other second sputtering cathode, the second sputtering cathode is in an idle state and kept from being sputtered in the preparation of the nano-multilayer film.

In some examples, the first sputtering cathode of the magnetron sputtering coating device is the graphite target, while the second sputtering cathode is a titanium target. First, the substrate to be coated (or the product to be coated) is put on the work steady in the deposition chamber, and the deposition chamber is vacuumed to 2×10⁻⁴ Pa, with the high pure argon introduced at a flow of 25 sccm.

Then, the sputtering cathode can be cleaned, and all the sputtering cathodes can be applied with a current of 0.4 A and a voltage of 500 V for 20 minutes of sputter cleaning.

Then, the sputtering is performed. During the whole sputtering process, with the rotating stand rotated uniformly, the substrate to be coated can also be rotated on its axis.

More specifically, in the first sputtering stage, the first predetermined current value of the second sputtering cathode can be set at 3.0-7.0 A, voltage of the second sputtering cathode can be set at 800 V. The first predetermined bias voltage is 100-200 V, the first predetermined duration is 5-15 min.

Then, the working bias voltage can be set at the second predetermined of 60-130 V, wherein, the two first sputtering cathodes can be set at the initial working current 11 of 0.5-2.0 A. For the two first sputtering cathodes, at a time interval of such as 5-15 min, their working current is increased by a certain value such as 0.5-1.5 A, until their working current is increased to a predetermined value such as 4.0-7.0 A. On the contrary, for the one second sputtering cathode, at a time interval of 5-15 min, its working current is decreased by a certain value such as 0.5-1.5 A, until its working current is decreased to a predetermined value such as 0-3.0 A.

That is, during the sputtering process of the first stage, the current of the first sputtering cathode is increased stepwise, and the current of the second sputtering cathode is decreased stepwise, their increased and decreased amounts being possible to be identical or different, their time intervals being possible to be identical or different, their voltage being kept constant in the whole process;.

Thus, after a period of sputtering such as for 50-100 min, the transition layer of 300-500 nm in thickness composed of titanium film and a mixed layer of TiC and quasi-graphite can be deposited onto the product of the metal material. Because the current gradients of different targets are controlled in the process, the mass percentage of Ti in the transition layer, in proximity to the metal material layer upwards, is decreased gradually from a higher content, while the mass percentage of C is increased gradually from a lower content, thus the binding force with the substrate, especially the substrates of metal or alloy materials, being increased, also its lubricity being increased gradually.

It is necessary to explain that, in the present invention the mixed layer of titanium carbide and quasi-graphite refers to a layer of titanium carbide mixed with the quasi-graphite; that is, the mixed layer contains quasi-graphite and titanium carbide at the same time.

Then, in the second sputtering stage, the working current of the first sputtering cathode can be kept at 4.0-7.0 A, which is the last predetermined value of the first sputtering stage. Certainly, the working current of the first sputtering cathode can also be set and kept to be reset and kept at a desired predetermined value such as 4.0-7.0 A. Meanwhile, the working current of the second sputtering cathode is set and kept at a desired predetermined value such as 0.5-2.0 A, voltage of all sputtering cathodes is kept at constant voltage of 800 V. Sputtering is performed for 120-400 min, during which the working bias voltage is kept constant of the second predetermined bias voltage. Under these processing conditions, a composite layer of 1600-3300 nm is formed, which is a multilayer structure with the quasi-graphite layer (the content of *sp*² is about 60 %) and the diamond-like carbon layer (the content of *sp*³ is about 70 %) alternately laminated.

Then, in the third sputtering stage, the working bias voltage is kept at constant of the second predetermined bias voltage. The voltage and current of the second sputtering cathode are decreased to zero, making it idle, and the current of the two first sputtering cathodes is reset to a desired predetermined value such as 4.0-7.0 A, and can certainly also be kept at the current of the second sputtering stage. Voltage is kept at constant voltage of 800 V, and sputtering is performed for 15-20 min. Under these processing conditions, a diamond-like carbon top-layer film of about 100 nm is separately formed on the composite layer, the quasi-diamond of high hardness making the nano-multilayer film have better hardness and wear resistance. So far a nano-multilayer film in combination with the substrate is formed; in the example, the mass percentage of C in the formed nano-multilayer film is 70-97.6 %, and the mass percentage of Ti is 2.4-30 %.

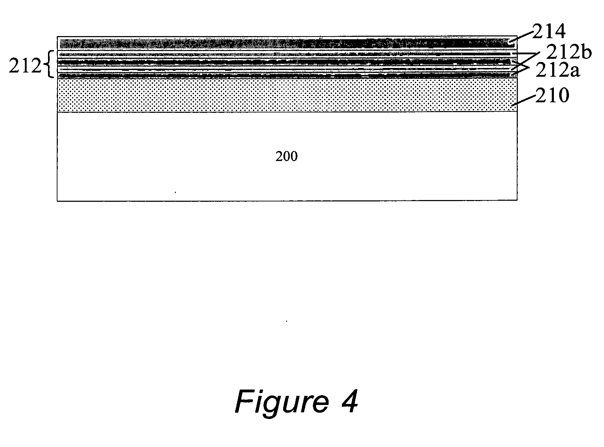

The nano-multilayer film of the present invention, formed by the above method with the above device that is in combination with the substrate, is shown in Fig. 4, which includes the transition layer 210 composed of Ti film and a mixed layer of TiC and quasi-graphite on the substrate 200, the composite layer 212 of alternately laminated diamond-like carbon and quasi-graphite on the transition layer 210, and the diamond-like carbon top-layer film 214 on the composite layer 212.The content of *sp*² in the quasi-graphite layer is 60 % and the content of *sp*³ in the diamond-like carbon layer is 70 %. In the direction from the substrate 200 to the diamond-like carbon top-layer film 214, the mass percentage of Ti in the mixed layer is gradually decreased, while the mass percentage of C is gradually increased. The composite layer 212 is a multilayer structure with the quasi-graphite layer 212a and the diamond-like carbon layer 212b alternately laminated. In the present invention, there is no limit to the number of the quasi-graphite layer and the diamond-like carbon layer in the composite layer 212. The interface of the nano-multilayer film where the transition layer is in combination with the substrate contains higher content of titanium and lower content of carbon, having a higher binding force with the substrate, also having low internal stress and good lubricity, thus the hardness and wear resistance being improved through the diamond-like carbon top-layer.

### Example 1

In a specific example 1, the two first sputtering cathodes are graphite targets, while the one second sputtering cathode is a titanium target. The arc between the second sputtering cathode and the first sputtering cathode in the sputtering device is substantially of 90°, the rotating stand has a rotational speed of 1.5 rpm, and the substrate to be coated is rotated on its axis. Before coating, the substrate to be coated is put onto the work steady of the deposition chamber, and the deposition chamber is vacuumed to 2×10⁻⁴ Pa, with the high pure argon introduced at a flow of 25 sccm.

Then, all the sputtering cathodes are applied with a current of 0.4 A and a voltage of 500 V for 20 minutes of sputter cleaning.

Then the sputtering is performed, graphite targets do not work first. Working bias voltage is set to 150 V, one titanium target being set to have a constant voltage of 800 V and a current of 7.0 A, and sputtering of 10 min is performed.

Then, the working bias voltage is decreased to 100 V. The titanium target is kept at constant voltage of 800 V and having a current decreased stepwise by 1.0 A at intervals of 10 min until down to zero. The two graphite targets are set to have constant voltage of 800 V and a working current started from 1.5 A and increased stepwise by 1.0 A at intervals of 10 min until up to 7.0 A. After sputtering of 60 min, a transition layer composed of titanium film and a mixed layer of TiC and quasi-graphite (the ratio of C of the mixed layer is increased gradually, while the ratio of Ti is decreased gradually) of 300 nm is deposited onto the substrate;.

Then, with the current and voltage of the two graphite targets kept constant, the current of the titanium target is increased to 1.0 A, with the voltage kept at constant voltage. The working bias voltage is still 100 V. After 120 min, a composite layer of 1600 nm is formed on the transition layer, comprising the quasi-graphite layer (the content of *sp*² is about 60 %) and the diamond-like carbon layer (the content of *sp*³ is about 70 %) alternately laminated.

Then the current and voltage of the titanium target are decreased to zero, that is to say does not work, and the current of the graphite target is increased to 7.0 A, with the voltage kept at 800 V. The working bias is kept at 100 V. fter 15 min, a diamond-like carbon film of 100 nm is formed on the composite layer. In the example, the mass percentage of C in the formed nano-multilayer film is 97 %, and the mass percentage of Ti is 3 %.

### Example 2

In another specific example 2, the two first sputtering cathodes in the sputtering device are graphite targets, while the one second sputtering cathode is a titanium target. The arc between the second sputtering cathode and the first sputtering cathode is substantially of 90°, the rotating stand has a rotational speed of 2.0 rpm, and the substrate to be coated is rotated on its axis. Before coating, the substrate to be coated is put onto the work steady of the deposition chamber, and the deposition chamber is vacuumed to 2×10⁻⁴ Pa, with the high pure argon introduced at a flow of 25 sccm.

Then, all the sputtering cathodes are applied with a current of 0.4 A and a voltage of 500 V for 20 minutes of sputter cleaning.

Then the sputtering is performed, graphite targets do not work first. Working bias voltage is set to 120 V. One titanium target is set to have a constant voltage of 800 V and a current of 6.5 A, and sputtering of 5 min is performed.

Then, working bias voltage is decreased to 110 V, and the titanium target is kept at constant voltage of 800 V and having a current decreased stepwise by 1.0 A at intervals of 5 min until down to zero. The two graphite targets are set to have constant voltage of 800 V and a working current started from 0.8 A and increased stepwise by 1.0 A at intervals of 5 min until up to 6.5 A. After 80 min, a transition layer composed of titanium film and a mixed layer of TiC and quasi-graphite (the ratio of C of the mixed layer is increased gradually, while the ratio of Ti is decreased gradually) of 400 nm is deposited onto the substrate.

Then, with the current and voltage of the two graphite targets kept constant, the current of the titanium target is increased to 1.5 A, with the voltage kept at constant voltage. The working bias voltage is still 110 V. After 180 min, a composite layer of 1800 nm is formed on the transition layer, comprising the quasi-graphite layer (the content of *sp*² is about 60 %) and the diamond-like carbon layer (the content of *sp*³ is about 70 %) alternately laminated.

Then the current and voltage of the titanium target are decreased to zero. The current of the graphite target is increased to 6.5 A, with the voltage kept at 800 V. The working bias is kept at 110 V. After 15 min, a diamond-like carbon film of 100 nm is formed on the composite layer. In the example, the mass percentage of C in the formed nano-multilayer film is 92 %, and the mass percentage of Ti is 8 %.

### Example 3

In still another specific example 3, the two first sputtering cathodes in the sputtering device are the graphite targets, while the one second sputtering cathode is a titanium target. The arc between the second sputtering cathode and the first sputtering cathode is substantially of 90°, the rotating stand has a rotational speed of 2.5 rpm, and the substrate to be coated is rotated on its axis. Before coating, the substrate to be coated is put onto the work steady of the deposition chamber, and the deposition chamber is vacuumed to 2×10⁻⁴ Pa, with the high pure argon introduced at a flow of 25 sccm.

Then, all the sputtering cathodes are applied with a current of 0.4 A and a voltage of 500 V for 20 minutes of sputter cleaning.

Then the sputtering is performed, graphite targets do not work first. Working bias voltage is set to 150 V. One titanium target is set to have a constant voltage of 800 V and a current of 6.0 A, and sputtering of 12 min is performed.

Then, a working bias voltage is decreased to 120 V, and the titanium target is kept at constant voltage of 800 V and having a current decreased stepwise by 1.0 A at intervals of 12 min until down to zero. The two graphite targets are set to have constant voltage of 800 V and a working current started from 1.8 A and increased stepwise by 1.0 A at intervals of 12 min until up to 6.0 A. After 100 min, a transition layer composed of titanium film and a mixed layer of TiC and quasi-graphite (the ratio of C of the mixed layer is increased gradually, while the ratio of Ti is decreased gradually) of 500 nm is deposited onto the substrate.

Then, with the current and voltage of the two graphite targets kept constant, the current of the titanium target is increased to 1.8 A, with the voltage kept at constant voltage. The working bias voltage is still 120 V. After 260 min, a composite layer of 1900 nm is formed on the transition layer, comprising the quasi-graphite layer (the content of *sp*² is about 60 %) and the diamond-like carbon layer (the content of *sp*³ is about 70 %) alternately laminated.

Then the current and voltage of the titanium target are decreased to zero. The current of the graphite target is increased to 6.0 A, with the voltage kept at 800 V. Working bias is kept at 120 V. After 15 min, a diamond-like carbon film of 100 nm is formed on the composite layer. In the example, the mass percentage of C in the formed nano-multilayer film is 86 %, and the mass percentage of Ti is 14 %.

An observation is made to the surface of the nano-multilayer film of the above specific examples 1-3 by the scanning electron microscope, finding that the surface of the nano-multilayer film has small surface roughness. The hardness of the nano-multilayer film is measured by a nanoindentation instrument, with a load of 10 mN, the pressed depth being greater than 10 times of the surface roughness and smaller than 1/10 of the thickness of the nano-multilayer film, so as to guarantee the authenticity and validity of the measured hardness. The longitudinal binding force and the lateral binding force of the nano-multilayer film are evaluated through the standard indentation instrument and the automatic scratch test. With the load of the indentation test being 150 N and the radius of the indenter being 0.2 mm, no crack and delamination is found around the indentation, indicating that the nano-multilayer film has a high longitudinal binding force. The load is increased gradually from 10 N to 85 N, with a sliding velocity of 10 mm/min. There is no crack and peeling around and at the edge of the scratch, indicating that the nano-multilayer film has a high lateral binding force.

The ball-disc friction and wear machine is used for determination of the friction and wear properties of the nano-multilayer film. The grinding ball is a Si₃N₄ ceramic ball having a hardness of 1500 HV and a diameter of 3 mm. The load is 10 N, the sliding velocity is 0.10 m/s, and the friction duration is 30 min. The experiments are made under the conditions of room temperature, no lubrication, and humid air (50 % relative humidity). In the testing process, the change of the friction coefficient is automatically recorded. After the test, the wear volume is measured with a step meter, with the wear rate calculated. Table 1 shows the hardness, average friction coefficient and wear rate of the nano-multilayer film of Examples 1, 2 and 3.

**Table 1**

| Nano-multilayer film | Content of Ti (at.%) | Sectional binding force of nano-multilayer film with substrate (N) | | Average friction coefficient (*µ*) | Wear rate (m³/Nm) |
|---|---|---|---|---|---|
| | | Longitudinal | Lateral | | |
| Example 1 | 3 | >150 | ≧95 | 0.048 | 2.8×10⁻¹⁷ |
| Example 2 | 8 | >150 | ≧95 | 0.052 | 2.3×10⁻¹⁷ |
| Example 3 | 14 | >150 | ≧5 | 0.046 | 3.1×10⁻¹⁷ |

The nano-multilayer film of the present invention has a low friction coefficient, super wear resistance and a high bonding force with the substrate of the product. In the nano-multilayer film of the specific examples 1, 2 and 3, the average friction coefficients of their films are 0.048, 0.052 and 0.046, respectively, without obvious change of the friction coefficient during the test process, indicating that the nano-multilayer film has good friction stability, the surface wear trace of the nano-multilayer film being very shallow after the wear; the wear rates of the nano-multilayer films of the specific examples 1, 2 and 3 are 2.8×10⁻¹⁷, 2.3×10⁻¹⁷ and 3.1×10⁻¹⁷ m³/Nm, respectively, indicating that the nano-multilayer film has very good wear resistance. Moreover, the pits of the nano-multilayer films of the specific examples 1, 2 and 3 formed under the load of 150 N have no crack, delamination or peeling trace at the edge thereof; under the load of 85 N, there is no crack or peeling of the nano-multilayer film observed in the area and at the edge of the scratch formed on the surface of the nano-multilayer film. The pit and scratch tests show that the nano-multilayer film has excellent bonding strength with the metal substrate.

## Claims

1. A nano-multilayer film comprising:
a composite layer (212), which is a multilayer structure with a quasi-graphite layer (212a) that also includes titanium, and a diamond-like carbon layer (212b) alternately laminated, where the *sp*² content in the quasi-graphite layer is 60 % and the *sp*³ content in the diamond-like carbon layer is 70 %; and
a diamond-like carbon top-layer film (214) on the composite layer;
**characterized in that** the film further comprises:
a transition layer (210) in combination with a substrate (200) below the composite layer, which transition layer includes a pure titanium film on the substrate and a mixed layer of titanium carbide and quasi-graphite on the titanium film, and
in the direction from the substrate to the diamond-like carbon top-layer film, the mass percentage of Ti in the mixed layer of titanium carbide and quasi-graphite is gradually decreased, while the mass percentage of C is gradually increased.

2. The nano-multilayer film according to claim 1, **characterized in that** the transition layer has a thickness of 300-500 nm.

3. The nano-multilayer film according to claim 1, **characterized in that** the composite layer has a thickness of 1600-3300 nm.

4. The nano-multilayer film according to claim 1, **characterized in that** the diamond-like carbon top-layer film has a thickness of 100-150 nm.

5. The nano-multilayer film according to claim 1, **characterized in that** the mass percentage of C in the nano-multilayer film is 70-97.6 %, and the mass percentage of Ti is 2.4-30 %.

## Patentansprüche

1. Eine Nano-Mehrschichtfolie bestehend aus:
einer Verbundschicht (212), bei der es sich um eine Mehrschichtstruktur mit einer Quasigraphitschicht (212a), die auch Titan enthält, und einer diamantartigen Kohlenstoffschicht (212b) handelt, die abwechselnd aufeinander laminiert sind, wobei der sp2-Anteil in der Quasigraphitschicht 60 % beträgt und der sp3-Anteil in der diamantartigen Kohlenstoffschicht 70 %; und einer diamantartigen Kohlenstoffoberfolie (214) auf der Verbundschicht;
**dadurch gekennzeichnet, dass** die Folie weiterhin aus Folgendem besteht:
einer Zwischenschicht (210) in Kombination mit einem Substrat (200) unterhalb der Verbundschicht, wobei die Zwischenschicht auf dem Substrat eine reine Titanfolie enthält und auf der Titanfolie eine Mischschicht aus Titancarbid und Quasigraphit liegt, und
in Richtung vom Substrat hin zur diamantartigen Kohlenstoffoberfolie der prozentuale Masseanteil von Ti in der Mischschicht aus Titancarbid und Quasigraphit allmählich abnimmt, während der prozentuale Masseanteil von C allmählich zunimmt.

2. Die Nano-Mehrschichtfolie aus Patentanspruch 1, **dadurch gekennzeichnet, dass** die Zwischenschicht 300-500 nm dick ist.

3. Die Nano-Mehrschichtfolie aus Patentanspruch 1, **dadurch gekennzeichnet, dass** die Verbundschicht 1.600-3.300 nm dick ist.

4. Die Nano-Mehrschichtfolie aus Patentanspruch 1, **dadurch gekennzeichnet, dass** die diamantartige Kohlenstoffoberfolie 100-150 nm dick ist.

5. Die Nano-Mehrschichtfolie aus Patentanspruch 1, **dadurch gekennzeichnet, dass** der prozentuale Masseanteil von C in der Nano-Mehrschichtfolie 70-97,6 % beträgt, und der prozentuale Masseanteil von Ti 2,4-30 %.

## Revendications

1. Nano-film multicouches comprenant :
une couche composite (212) se présentant comme une structure multicouches avec une couche de quasi graphite (212a), comprenant également du titane, et une couche de carbone de type diamant (212b), stratifiée de façon alternée, la teneur en *sp²* de la couche de quasi graphite étant 60 % , et la teneur en *sp*³ de la couche de carbone de type diamant étant 70 % ; et un film à couche supérieure en carbone de type diamant (214) sur la couche composite ;
**caractérisé en ce que** le film comprend en outre :
une couche de transition (210) combinée avec un substrat (200) sous la couche composite, ladite couche de transition comprenant un film de titane pur sur le substrat, et une couche mixte de carbure de titane et de quasi graphite sur le film de titane, et
dans la direction du substrat au film à couche supérieure en carbone de type diamant, le pourcentage en masse de Ti dans la couche mixte de carbure de titane et de quasi graphite diminuant progressivement tandis que le pourcentage en masse de C augmente progressivement.

2. Le nano-film multicouches selon la revendication 1, **caractérisé en ce que** l'épaisseur de la couche de transition est comprise entre 300 et 500 nm.

3. Le nano-film multicouches selon la revendication 1, **caractérisé en ce que** l'épaisseur de la couche composite est comprise entre 1 600 et 3 300 nm.

4. Le nano-film multicouches selon la revendication 1, **caractérisé en ce que** l'épaisseur de la couche supérieure en carbone de type diamant est comprise entre 100 et 150 nm.

5. Le nano-film multicouches selon la revendication 1, **caractérisé en ce que** le pourcentage en masse de C dans le nano-film multicouches est compris entre 70 et 97,6 %, et le pourcentage en masse de Ti est compris entre 2,4 et 30 %.
